Europäisches Patentamt

European Patent Office

Office européen des brevets

(19)

(11) Veröffentlichungsnummer : **0 318 777 B1**

(12) **EUROPÄISCHE PATENTSCHRIFT**

(45) Veröffentlichungstag der Patentschrift :
29.03.95 Patentblatt 95/13

(51) Int. Cl.$^6$ : **G01N 33/52,** G01N 33/543,
G01N 33/551, G01N 33/552

(21) Anmeldenummer : 88119276.9

(22) Anmeldetag : 19.11.88

(54) **Testträger zur Analyse einer Probenflüssigkeit und Verfahren zu seiner Herstellung.**

(30) Priorität : 28.11.87 DE 3740471

(43) Veröffentlichungstag der Anmeldung :
07.06.89 Patentblatt 89/23

(45) Bekanntmachung des Hinweises auf die
Patenterteilung :
29.03.95 Patentblatt 95/13

(84) Benannte Vertragsstaaten :
AT BE CH DE ES FR GB GR IT LI LU NL SE

(56) Entgegenhaltungen :
EP-A- 0 016 387
EP-A- 0 133 895
EP-A- 0 267 519
US-A- 4 560 504

(73) Patentinhaber : BOEHRINGER MANNHEIM
GMBH
D-68298 Mannheim (DE)

(72) Erfinder : Eikmeier, Heino, Dr.
Bismarckstrasse 8
D-6143 Lorsch (DE)
Erfinder : Freitag, Helmut, Dr.
9115 Hague Road
Indianapolis, IN 46250 (US)
Erfinder : Münter, Karin, Dr.
Karl-Ladenburg-Strasse 22
D-6800 Mannheim 1 (DE)
Erfinder : Pollmann, Klaus, Dr.
9115 Hague Road
Indianapolis, IN 46250 (US)
Erfinder : Wilk, Hans-Erich, Dr.
Goldregenstrasse 5
D-6143 Lorsch (DE)
Erfinder : Winkle, Johannes
Vordergasse 24
D-6945 Hirschberg-Leutershausen (DE)

(74) Vertreter : Pfeifer, Hans-Peter, Dr.rer.nat.
Patentanwalt
Nowackanlage 15
D-76137 Karlsruhe (DE)

**Beschreibung**

Die Erfindung betrifft einen mehrschichtigen Testträger zur Analyse einer Probenflüssigkeit.

Zur qualitativen oder quantitativen analytischen Bestimmung von Bestandteilen von Flüssigkeiten werden in jüngerer Zeit zunehmend sogenannte "trägergebundene Tests" verwendet. Bei diesen sind Reagenzien in eine oder mehrere Testschichten eines festen Testträgers eingebettet, der mit der Probe in Kontakt gebracht wird. Die Reaktion von Probe und Reagenzien führt zu einem nachweisbaren Signal, insbesondere einem Farbumschlag, welcher visuell oder mit Hilfe eines Gerätes, meistens reflexionsphotometrisch, ausgewertet werden kann. Vor allem zur Analyse von Körperflüssigkeiten (z.B. Blut und Urin) für medizinische Zwecke wird die Testträger-Analytik verwendet.

Testträger sind häufig als Teststreifen ausgebildet, die im wesentlichen aus einer länglichen Basisschicht aus Kunststoffmaterial und darauf angebrachten Testschichten bestehen. Es sind jedoch auch Testträger bekannt, die als quadratische oder rechteckige Plättchen ausgebildet sind.

Die Erfindung richtet sich insbesondere auf Testträger für immunologische Bestimmungen, die in der medizinischen Analytik von besonderer Bedeutung sind. Diese Analayseverfahren sind allgemein dadurch gekennzeichnet, daß der Analyseablauf hochspezifische Bindungsreaktionen zwischen immunologischen Bindungspartnern, insbesondere zwischen Antigenen und Antikörpern oder zwischen Haptenen und Antikörpern einschließt. Soweit immunologische Bestimmungen mit Hilfe von Testträgern durchgeführt werden sollen, ist es häufig erforderlich, einen der Bindungspartner innerhalb einer Testschichtstruktur zu fixieren, wobei schwierige Anforderungen zu erfüllen sind.

Eine übersicht über bekannte Testschichtstrukturen für immunologische Bestimmungen ist beispielsweise der DE-A-33 29 728 zu entnehmen. Danach läßt sich das leichte Eindringen der Probe in die Schicht und die erforderliche freie Berührung durch Verwendung einer entsprechend offenporigen porösen Struktur realisieren. Genannt werden beispielsweise Membranfilter, Gewebe, Filterpapiere und Glasfaserfilterschichten. Die Liganden können an kleinen Pdlymerkügelchen gebunden sein, die in der porösen Struktur angeordnet sind. Zu diesem Zweck werden durch Anätzen des Polymers mit Hilfe einer Säure funktionelle Gruppen gebildet.

In der EP-A-13 156 ist eine Testschichtstruktur, u.a. für immunologische Anwendungen beschrieben, bei der die Polymerkügelchen mit Hilfe eines Klebemittels in Form eines von den Teilchen verschiedenen organischen Polymersisats zu einer dreidimensionalen Gitterstruktur verklebt sind. Auf die Offenporigkeit der so erhaltenen Struktur wird besonderer Wert gelegt.

Die Erfindung richtet sich insbesondere auf eine wichtige Teilgruppe von immunologischen oder sonstigen auf einer spezifischen Bindungsreaktion basierenden Testträgern, nämlich solche die auf dem sogenannten IEMA-Testprinzip basieren. Solche Testträger weisen im Strömungsweg der Probenflüssigkeit hintereinander folgende Testschichten auf:

- Eine Konjugatschicht, die ein Konjugat eines Bindungspartners der spezifischen Bindungsreaktion enthält, welches von der Probenflüssigkeit gelöst wird, wobei Komplexe gebildet werden, die ein Maß für den in der Probe enthaltenen Analyt sind und
- eine Trennschicht, die eine poröse Struktur hat und einen Liganden enthält, der eine spezifische Bindungsreaktion mit dem Konjugat macht, um überschüssiges Konjugat aus der in die Trennschicht eindringenden Probenflüssigkeit abzutrennen und die Komplexe, die ein Maß für den in der Probe enthaltenen Analyt sind, passieren zu lassen.

Bezüglich des Ablaufs eines IEMA-Tests auf einem Testträger wird ergänzend auf die europäische Patentanmeldung 0 267 519, insbesondere Spalte 8, Bezug genommen.

Der Erfindung liegt die Aufgabe zugrunde, bei einem auf dem IEMA-Testprinzip basierenden Testträger mit den vorstehend bezeichneten Merkmalen eine Trennschicht mit sehr guter Wirksamkeit bei geringer Schichtdicke zur Verfügung zu stellen und dadurch Analysen mit hoher Qualität bei einem sehr geringen Probevolumen zu ermöglichen.

Die Aufgabe wird durch einen Testträger mit den Merkmalen des Anspruch 1 gelöst.

Die Trennschicht wird erfindungsgemäß nach einem mindestens dreistufigen Verfahren hergestellt. In der ersten Stufe wird der Ligand mit Hilfe der Kopplungsgruppen an den Trägerpartikeln fixiert. In der zweiten Stufe werden die Partikel durch Filtrieren, Zentrifugieren oder Sedimentieren von den zur Fixierung des Reagenz notwendigen Substanzen getrennt. Schließlich werden in der dritten Stufe entweder die Trägerpartikel in eine vorher vorhandene poröse Trägermatrix inkorporiert oder die Schichtstruktur mit den Trägerpartikeln wird als Film hergestellt, der durch Zusatz eines öffners in Form von korpuskulären Bestandteilen geöffnet ist.

Techniken zur Ankopplung von Liganden verschiedener Art, insbesondere auch von Antikörpern, sind für andere Anwendungszwecke bekannt, beispielsweise für die Herstellung von Füllmaterialien für Chromatographiesäulen, mit deren Hilfe immunologische Reagenzien gereinigt werden können. Die reaktiven organischen Gruppen können insbesondere Amingruppen oder carboxylgruppen sein, an die der Ligand mit Hilfe einer Amidbindung angekoppelt wird. Eine derartige Kopplung für Antikörper wird in mehreren Varianten in der US-A-45 60 504 beschrieben. Die Technik basiert auf analogen Verfahren zur Bindung von Enzymen auf anorganischen Trägern, wie sie in den Artikeln Howard H. Weetall "Enzymes immobilized on inorganic supports", Trends in Biotechnology, 1985, PP. 276 - 280 und H. H. Weetall "Covalent coupling methods for inorganic support materials" in "Methods in Enzymology" Vol. X L IV Ed. K. Mosbach, Academic Press, New York, San Fransico, London 1976, pp. 134 - 149 beschrieben werden.

Die Verwendung anorganischer Partikel in Testschichten von Testträgern ist in anderen Sachzusammenhängen bekannt. Beispielsweise wird in der EP-A-0 016 387 eine Testschichtstruktur beschrieben, bei der verschiedene solche Materialien als Öffner (vorzugsweise Diatomeenerde-Teilchen) oder Pigment zur Verbesserung der Remissionseigenschaften (vorzugsweise $TiO_2$) eingesetzt werden.

Erfindungsgemäß erzeugt man die Kopplungsgruppen an der Oberfläche der anorganischen Teilchen bevorzugt mit Silanisierungsreagenzien, beispielsweise 3-Aminopropyl-Triaethoxysilan deren Silangruppen unter Abspaltung von $C_2H_5OH$ an die anorganischen Materialien ankondensieren (vgl. die zitierten Artikel).

Als Trägerpartikel können zweckmäßigerweise $SiO_2$-Teilchen verwendet werden, wie sie beispielsweise als Silica-Gele für chromatographische Zwecke angeboten werden. Besonders bevorzugt ist jedoch die Verwendung von Titandioxid.

Gemäß einer vorteilhaften Variante ist der Ligand über Brückenmoleküle an die Kopplungsgruppen gebunden. Als Brückenmoleküle werden dabei organische Moleküle bezeichnet, die sich eignen, den Abstand zwischen dem Liganden und der Teilchenoberfläche zu vergrößern. Sie müssen mindestens zwei reaktive organische Gruppen aufweisen, die zweckmäßigerweise in der Nähe der Enden der Moleküle liegen. Mit der einen reaktiven organischen Gruppe werden sie an die Kopplungsgruppen kovalent gebunden, die andere reaktive organische Gruppe dient der kovalenten Bindung des Liganden. Im übrigen sollen die Brückenmoleküle abgesättigt sein, wobei jedoch auf der Ligandenseite auch mehrere reaktive organische Gruppen zur Ankopplung von mehreren Liganden vorgesehen sein können.

Nach einer besonders bevorzugten Ausführungsform haben die Trägerpartikel einen mittleren Durchmesser zwischen 0,2 µm und 1µm , wobei auch hier Titandioxid besonders geeignet ist.

Es besteht aus weitgehend homogenen Partikeln mit einer definierten Oberfläche. Außerdem hat es Pigmenteigenschaften. Durch diese Doppelfunktion ergibt sich eine Reduzierung der Schichtdicke bei gleichbleibender Wirksamkeit der Schicht.

Die Erfindung ermöglicht eine hohe und gleichmäßige Beladung der Testschicht mit dem Liganden. Damit ergibt sich eine ausgezeichnete Wirksamkeit in dem jeweiligen Analyseverfahren bei gleichzeitig geringer Schichtdicke. Die geringe Schichtdicke wiederum ermöglicht es, mit einer sehr kleinen Probenmenge auszukommen.

Auch herstellungstechnisch bringt die Erfindung erhebliche Vorteile. Insbesondere können die Trägerpartikel mit organischen Lösungsmitteln in Kontakt gebracht werden, ohne Schaden zu nehmen. Außerdem lassen sie sich ausgezeichnet durch Filtrieren, Zentrifugieren oder Sedimentieren von den zur Fixierung des Liganden erforderlichen Reagenzien trennen.

Die anorganischen Trägerpartikel lassen sich in verschiedene ausreichend offenporige Matrixmaterialien inkorporieren, wie sie beispielsweise in der eingangs zitierten DE-A-33 29 728 beschrieben werden. Besonders bevorzugt wird jedoch eine Testschicht verwendet, die aus einer Lösung oder Dispersion filmbildender organischer Kunststoffe unter Zusatz eines Öffners in Form von korpuskulären Bestandteilen hergestellt ist. Hierzu wird auf die EP-B-16 387 Bezug genommen. Eine derartige Testschicht ist im Gegensatz zu Geweben und Vliesen formstabil.

Bevorzugt besteht der Öffner aus anorganischen Partikeln mit einem mittleren Teilchendurchmesser von mindestens 5 µm. Ein bevorzugtes Material ist Siliciumdioxid. Besonders bevorzugt wird Diatomeenerde als Öffner verwendet.

Gemäß einer weiteren bevorzugten Ausführungsform wird nur eine verhältnismäßig geringe Menge an filmbildenden organischen Kunststoffen verwendet, die maximal 25 Gew.% der Gesamtmenge von Trägerpartikeln und Öffnerpartikeln beträgt. Die Gewichtsrelation zwischen Trägerpartikeln und Öffner liegt vorzugsweise zwischen 2:1 und 1:2. Damit ergeben sich in dem Dreikomponentensystem aus Film, Trägerpartikeln und Öffner optimale Verhältnisse bezüglich der Beladungsdichte mit dem Liganden, leichter Zugänglichkeit des fixierten Liganden für die in die Schicht eintretende Probe und geringe Schichtdicke.

Die Erfindung wird im folgenden anhand von in den Figuren dargestellten Ausführungsbeispielen näher erläutert; es zeigen:

Fig. 1 und Fig. 2      die Ankopplung eines Liganden über Brückenmoleküle an die Trägerpartikel in einer symbolischen Darstellungsweise

Fig. 3      einen erfindungsgemäßen Testträger in Seitenansicht

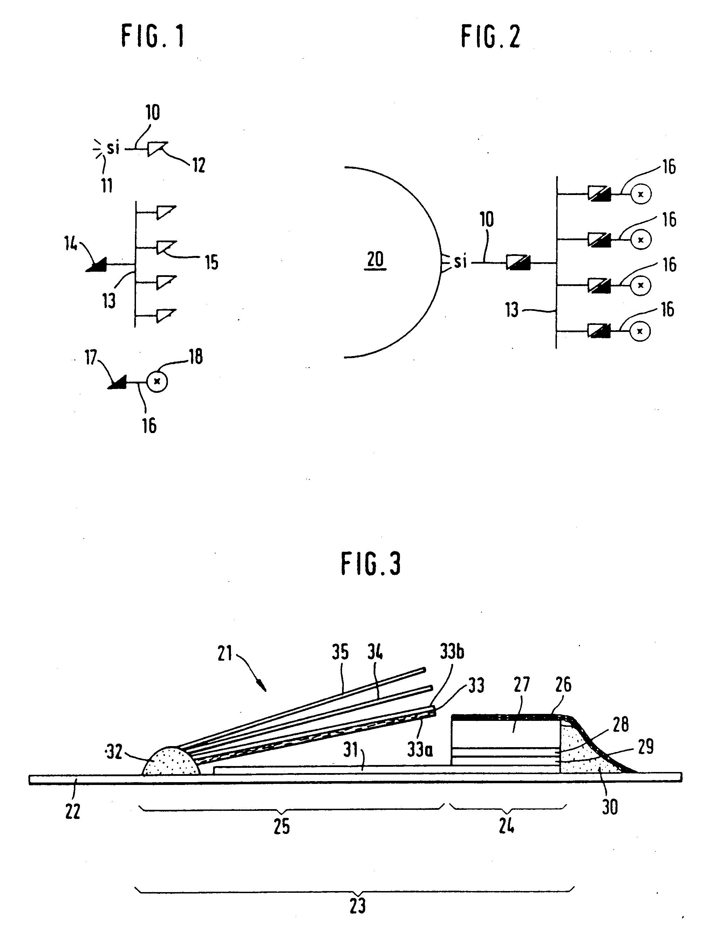

Fig. 1 verdeutlicht die in Fig. 2 verwendete Symbolik. Das Silanisierungsreagenz 10 enthält eine Silangruppe 11 und eine reaktive organische Gruppe 12, die beispielsweise eine Carboxylgruppe oder ein Amingruppe sein kann und als Kopplungsgruppe dient.

Das mit 13 bezeichnete Brückenmolekül hat einerseits eine erste trägerpartikelseitige reaktive organische Gruppe 14 zur Ankopplung an die komplimentäre Gruppe 12 des Silanisierungsreagenz. Andererseits hat das Brückenmolekül 13 ligandenseitige reaktive organische Gruppen 15 zur Ankopplung des Liganden. Es muß mindestens eine ligandenseitige reaktive organische Gruppe 15 vorhanden sein. Bevorzugt sind jedoch mehrere solche Gruppen vorhanden, um das Ankoppeln mehrerer Liganden an ein Brückenmolekül zu ermöglichen und damit die Beladungsdichte zu erhöhen.

Der Ligand 16 hat eine reaktive organische Gruppe 17, die zu den ligandenseitigen reaktiven organischen Gruppen 15 des Brückenmoleküls 13 komplementär ist und so die kovalente Kopplung ermöglicht. Mit 18 ist das an der Reaktion, insbesondere einer hochspezifischen immunologischen Bindungsreaktion teilnehmende Ende des Liganden 16 bezeichnet. Für den Fall, daß der Ligand 16 ein Antigen ist, symbolisiert 18 das Epitop, an das ein "passender" Antikörper ankoppeln kann (antigene Determinante).

Fig. 2 zeigt symbolisch die Anordnung der Komponenten an einem Trägerpartikel, beispielsweise einem Titandioxydpartikel 20. Man erkennt, daß die Verwendung des Brückenmoleküls 13 zu einer bedeutenden Erhöhung der Beladungsdichte führt. Dies resultiert nicht nur aus der Tatsache, daß ein Brückenmolekül 13 ligandenseitig mehrere reaktive organische Gruppen 15 haben kann. Im Rahmen der Erfindung hat sich gezeigt, daß eine Erhöhung der Beladungsdichte auch erreicht wird, wenn das Brückenmolekül nur eine einzige solche Gruppe hat. Dies dürfte darauf zurückzuführen sein, daß insbesondere bei Verwendung sehr kleiner Trägerpartikel die räumlichen Verhältnisse weniger günstig sind, wenn man den Liganden mit seiner reaktiven organischen Gruppe 17 unmittelbar an die entsprechende komplementäre organische Gruppe 12 des Silanisierungsreagenz 10 ankoppelt.

Der in Fig. 3 dargestellte Testträger 21 hat die prinzipielle Form eines Teststreifens. Es handelt sich jedoch um ein mit den früheren Teststreifen kaum mehr vergleichbares hochwertiges Analysesystem, insbesondere für die Durchführung immunologischer Bestimmungen.

Auf einer Basisschicht 22 befindet sich der insgesamt mit 23 bezeichnete Testbereich, der sich nur über einen Teil der Länge der Basisschicht 22 erstreckt. Der Testbereich 23 läßt sich in eine Probenaufgabezone 24 und in eine Auswertezone 25 unterteilen.

In der Probenaufgabezone 24 erkennt man von oben nach unten ein Abdecknetz 26, eine Erythrozytenabtrennschicht 27 und zwei Reagenzschichten 28 und 29, die mit einem Schmelzkleberstreifen 30 an der Basisschicht 22 befestigt sind.

Eine Flüssigkeitstransportschicht 31 aus einem saugfähigen Material, die ebenfalls mit dem Schmelzkleberstreifen 30 fixiert ist, erstreckt sich aus der Probenaufgabezone 24 in die Auswertezone 25. Über dem nicht von den Schichten 26 bis 29 bedeckten Bereich der Flüssigkeitstransportschicht 31 befinden sich drei Schichten, die mit einem Schmelzkleberstreifen 32 an der Basisschicht 22 derartig befestigt sind, daß sie ohne äußeren Druck schräg von dieser abstehen und sie nicht berühren. Es handelt um eine Testschicht 33 mit einem erfindungsgemäß fixierten Liganden, um eine dritte Reagenzschicht 34 und um eine Abdeckfolie 35.

Der dargestellte bevorzugte Testträger ist insbesondere zur Durchführung immunologischer Bestimmungen geeignet, die dem sogenannten IEMA-Prinzip nachgebildet sind.

Soll beispielsweise ein in der Probe enthaltenes Antigen (Ag) bestimmt werden, so läuft die Analyse mit dem in Fig. 3 dargestellten Testträger folgendermaßen ab:

Ein Tropfen Blut (etwa 30 $\mu$ l) wird oberhalb der Erythrozytenabtrennschicht auf das Abdecknetz 26 aufgegeben und durchdringt die Erythrozytenabtrennschicht 27, die beispielsweise gemäß dem US-Patent 4,477,575 ausgebildet sein kann. Das so erhaltene Serum dringt in die Schicht 29 ein.

Die Schicht 29 enthält einen enzymatisch markierten Antikörper (Ak) für das Ag im Überschuß gegenüber der maximalen Ag-Konzentration in der Probe. Dieses Antikörper-Enzym Konjugat (AkE) wird von dem eindringenden Serum gelöst. Dabei bilden sich Komplexe zwischen dem AkE und dem Ag, die mit Ag-AkE bezeichnet werden. Da das AkE im Überschuß vorhanden ist, bleibt bei Einstellung des Gleichgewichts freies Konjugat AkE übrig.

Aufgabe der Schicht 33 ist es, dieses den weiteren Nachweis störende AkE durch eine immunologische Bindung zu beseitigen. Sie wird deshalb auch als immunologische Trennschicht bezeichnet. Sie enthält den Analyt oder ein Analytanalogon in trägerfixierter Form, wobei die Trägerfixierung erfindungsgemäß mit Hilfe von anorganischen Trägerpartikeln erfolgt.

Nach Ablauf einer vorbestimmten Inkubationszeit, in der sich das Gleichgewicht in der vorausgehenden Reaktion eingestellt hat, wird von oben ein Druck auf die Schichten 33 - 35 ausgeübt. Dies kann manuell oder - wie beispielsweise in der EP-A-129 220 beschrieben - mit Hilfe eines Gerätebauteils mechanisch geschehen. Durch das Andrücken kommt die immunologische Abtrennschicht 33 mit der Flüssigkeitstransportschicht 31 in Kontakt und die darin enthaltenen Bestandteile dringen in die Schicht 33 vor. Dabei koppeln die nicht komplexierten AkE an das fixierte Ag an, während die Ag-AkE Komplexe ungehindert weiter vordringen können.

Die Reagenzschicht 34 enthält ein farbbildendes Substrat für das Markierungsenzym. Wenn die Flüssigkeit das Substrat erreicht hat, katalysiert das Enzym der freien Ag-AkE Komplexe die Farbreaktion des Substrats. Die Änderungsgeschwindigkeit des Farbumschlages ist deswegen ein Maß für die bei der Reagenzschicht 34 angelangten freien Komplexe Ag-AkE. Diese wiederum sind ein Maß für das in der Probe enthaltene Ag. Bezüglich des Ablaufs eines IEMA Tests auf einem Testträger wird ergänzend auf die deutsche Patentanmeldung 36 38 654 Bezug genommen.

Für die Funktion des Testträgers nach Fig. 3 sind der Aufbau und die Wirkung der immunologischen Abtrennschicht 33 von besonderer Bedeutung. Die Farbänderung in der Substratschicht 34 ist nur dann ein reproduzierbares Maß für die Ag-Konzentration, wenn die Schicht 33 für eine vollständige Abtrennung der nicht komplexierten AkE sorgt. Wäre dies nicht der Fall, so wäre die enzymatische Aktivität im Bereich der Substratschicht 33 zu hoch und das Ergebnis würde verfälscht. Ähnliches gilt auch bei anderen immunologischen Bestimmungen bei denen deshalb ein Ligand mit möglichst hoher Ladungsdichte vorliegen soll.

Die Schicht 33 besteht vorzugsweise aus einem tragenden Gewebe oder Vlies 33a mit einer darauf angebrachten Beschichtung 33b, die vorzugsweise als Lösung oder Dispersion filmbildender organischer Kunststoffe unter Zusatz eines Öffners in Form von korpuskulären Bestandteilen ausgebildet ist, wie sie oben näher erläutert wurde. Diese Gewebeverbundschicht wird hergestellt, indem man unmittelbar auf das Gewebe oder Vlies die Filmschicht beschichtet, wobei die Viskosität der Filmschicht so eingestellt ist, daß sie zwar einerseits in das Gewebe eindringt, andererseits aber überwiegend auf einer Seite des Gewebes verbleibt. Hierzu wird auf die EP-B 113 896 Bezug genommen.

Durch die erfindungsgemäß mögliche ungewöhnlich hohe Beladungsdichte hat die Schicht 33 eine hervorragende Abtrennwirkung. Sämtliche nicht komplexierten AkE werden beim Eindringen in die Schicht sofort an das fixierte Ag gebunden, so daß die "störende" Enzymaktivität nur in der Nähe der unteren (d.h. der Flüssigkeitstransportschicht 31 zugewandten) Oberfläche der Schicht 33 verbleibt.

Dadurch gelangen, wie oben beschrieben, nur die Ag-AkE Komplexe zu dem Substrat in der Substratschicht 34. Im Regelfall wird das Substrat allerdings löslich sein und es ist deswegen nicht auszuschließen, daß eine Teilmenge des Substrats 34 durch Diffusion bis zu der unteren Oberfläche der Schicht 33 gelangt, wo das abgefangene AkE gebunden ist. Um eine Störung der Messung zu verhindern, enthält die Schicht eine ausreichende Menge an Pigment, so daß bei der visuellen oder apparartiven Auswertung des Farbumschlages von der Oberseite des Testträgers her ein solcher störender Farbumschlag nicht mit erfaßt wird.

Wenn als Trägerpartikel anorganische Teilchen verwendet werden, haben sie vielfach bereits Pigmenteigenschaften, so daß sie die Doppelfunktion eines Trägers für trägerfixierte Reagenzien und einer optischen Sperre erfüllen. Dies gilt vor allem für $TiO_2$. Dadurch ergibt sich eine Abtrennschicht, die schon bei sehr geringer Schichtdicke von weniger als 200 µm alle Anforderungen erfüllt.

Beispiele

1. Beispiel A: Gesamt-Thyroxin(T4)-Test
   1.1 Aufbau und Funktion des Testträgers
   Aufbau und Funktion des Testträgers entsprechend Figur 3. Um den Nachweis des gesamten im Serum vorliegenden Thyroxins zu ermöglichen, ist eine Freisetzung des proteingebundenen T4 durch ein geeignetes Verdrängungsreagenz erforderlich, welches sich in der ersten Reagenzschicht 28 befindet.
   1.2 Präparation der Abtrennschicht 33
   1.2.1 Amino-Silanisierung von $TiO_2$ ($TiO_2$-Si)
   50 g $TiO_2$ (RN43, Kronos-Titan, Leverkusen, Bundesrepublik Deutschland (BRD) werden in 1000 ml bidest. Wasser suspendiert und mit 10 ml 3-Aminopropyl -triethoxysilan (Sigma-Chemie, Deisenhofen, BRD) für 2 h bei 75°C unter Kontrolle des pH-Wertes (pH 3-4) gerührt. Danach wird über eine Glasfritte (G5) abgesaugt und mit bidest. Wasser bis zur Neutrale gewaschen.
   1.2.2 Aufbau der Brückenmoleküle ($TiO_2$-Si-GA-Cc)
   50 g silanisiertes Titandioxid ($TiO_2$-Si) werden in 250 ml Glutardialdehyd-Lösung (25 %ig in Wasser, Sigma-Chemie, Deisenhofen, BRD) suspendiert und bei pH 7,4 für 12 h gerührt. Danach wird über eine Glasfritte (G5) abgesaugt, zunächst mit bidest. Wasser und dann mit 0,5 M Phosphat-Puffer pH 7,6) gewaschen. Die feste Phase wird in 225 ml phat-Puffer aufgenommen und 12 h mit 25 ml Crotein-C-Lösung (10%ig in P-Puffer, Cc von Croda, Nettetal, BRD) gerührt. Zur Absättigung noch freier Aldehyd-Gruppen werden 50 g in P-Puffer gewaschene Festphase in 500 ml 0,5 M P-Puffer mit 0,5 M Glycin für 1 h gerührt. Nach intensivem Waschen (bidest. Wasser) wird das Sediment (~50g) in 450 ml Borat-Puffer (pH 8,5) aufgenommen und mit 50 ml NaCN-Borhydrid-Lösung (5 %ig in Borat-Puffer) für 15 min zur Reduktion der Schiffschen Basen gerührt. Anschließend wird über eine Glasfritte (G5) abgesaugt und mit P-Puffer und bidest. Wasser gewaschen.

1.2.3 Ankopplung von BOC-T4-Hydroxysuccinimid ($TiO_2$-Si-GA-Cc-T4) als Analyt-Analogon

10 g $TiO_2$-Si-GA-Cc werden in 200 ml 00,06 M $Na_2HPO_4$ (pH 8,8) suspendiert und mit 170 ml BOC-T4-Hydroxysuccinimid-Lösung 0,05 %ig in Dio xan) für 2 h im Dunkeln gerührt. Danach wird mit 0,06 M $Na_2HPO_4$/Dioxan (10:8,5) und mehrfach mit bidest. Wasser gewaschen und abgesaugt.

1.2.4 Filmbeschichtung auf Gewebe

Eine Beschichtungsmasse mit der Zusammensetzung:

```
Propiofan 70 D  (BASF, Ludwigshafen,BRD)      4  g

Brij 35 (Serva, Heidelberg,BRD)               1  g

        15 %ige  Lösung  in  Gal-Puffer

Polyox WSR 301 (Union Carbide, New York,USA)  5  g

        2,5 %ig  in  Gal-Puffer

TiO2-T4-Matrixkopplung,                       30  g

        40 %ige  Suspension  in  Gal-Puffer

Kieselgur MW 25                                9  g
                                              ─────
                                              49  g
```

Wird auf ein Gewebe PE 812 K[6] (Schweizerische Seidengazefabrik, Thal, Schweiz) mit einer Naßfilmdicke von 350 μm aufgetragen und getrocknet.

1.3 Präparation des Verdrängungsreagenz auf der ersten Reagenzschicht 28

200 mM T4-Verdrängungsreagenz werden in Gal-Puffer gelöst und auf Teebeutelpapier (Schöller und Hösch, Gernsheim, BRD) getränkt.

```
Gal-Puffer:    KH2PO4    10 mM
               MgCl2      5 mM
               NaCl      25 mM
               pH         7,0
```

1.4 Präparation der zweiten Reagenzschicht 29 als Konjugatschicht

Tränkung auf Gewebe PE 14 100 normal (Schweizer Seidengazefabrik, Thal,Schweiz). Zusammenseitzung der Tränklösung:

```
Hepes              50 mM
MgCl2               5 mM
Trehalose           1 %
Crotein C           1 %
<T4>-ßGal-Konjugat 80 U/ml
pH                  6,6
```

1.5 Präparation der dritten Reagenzschicht 34 als Substratschicht

Tränkung auf Gewebe PE HD-1 (Schweizer Seidengazefabrik, Thal, Schweiz) Zusammensetzung der Tränklösung: Chlorphenolrotgalactocid (CPRG) 20 mM in Gal-Puffer

1.6 Ergebnisse

30 μl Serum mit verschiedenen Gesamt-T4-Konzentrationen werden auf Teststreifen aufgetragen und in dem Gerät "Reflotron" der Anmelderin bei 567 nm vermessen:

6

| µg/dl T4 | % Remision nach 1 min |
|---|---|
| 0,55 | 52,25 |
| 6,65 | 44,51 |
| 10,00 | 38,52 |
| 20,90 | 31,48 |

Die erreichbare Abstufung im klinisch relevanten Bereich von 2,0 bis 17,0 µg/dl erlaubt eine sehr gute Meßgenauigkeit.

2. Beispiel B: Theophyllin-Test

2.1 Aufbau und Funktion des Testträgers

Wie Beispiel A, jedoch entfällt die erste Reagenzschicht 28.

2.2 Präparation der Abtrennschicht 33

2.2.1 Carboxy-Silanisierung von $TiO_2$ ($TiO_2$-Si) zur Erzeugung von Kopplungsgruppen

500 ml bidest. Wasser (mit Eisessig auf pH 5 eingestellt) werden bei 75° C mit 10 ml 3-Triaethoxysilylpropyl-Bernsteinsäureanhydrid (Wacker-Chemie, München, BRD) und 50 g $TiO_2$ (suspendiert in 200 ml bidest. Wasser) für 2 h gerührt. Danach wird über eine Glasfritte (G5) abgesaugt und mehrfach mit bidest. Wasser gewaschen.

2.2.2 Aufbau der Brückenmoleküle ($TiO_2$-Si-Cc)

25 g silanisiertes $TiO_2$ ($TiO_2$-Si) werden in 125 ml bidest. Wasser suspendiert. Bei pH 4,5 werden 1,5 g 1-Ethyl-3-(3-Dimethylaminopropyl)-Carbodiimid-HCl (Sigma-Chemie, Deisenhofen, BRD) zugesetzt und für 30 min bei Raumtemperatur gerührt. Danach werden 125 ml 2 %ige Crotein C-Lösung zugesetzt und für 12 h gerührt. Nach Zusatz von weiteren 50 ml Cc-Lösung wird noch 1 h weitergerührt und dann über eine Glasfritte (G5) mehrfach mit bidest. Wasser abgesaugt.

2.2.3 Ankopplung von Theophyllin-Polyhapten-Hydroxysuccinimid ($TiO_2$-Si-Cc-polyTheo) als Analyt-Analogon

15 g $TiO_2$-Si-Cc werden in 75 ml 0,1 M P-Puffer (pH 8,7) suspendiert und mit 150 mg aktiviertem Theophyllin-Polyhapten (poly-Theo-O-Su, gelöst in 75 ml DMSO) für 12 h gerührt. Danach wird über eine Glasfritte (G5) abgesaugt, 2x mit DMSO/bidest. Wasser (1:1) und mehrfach mit bidest. Wasser gewaschen.

2.2.4 Filmbeschichtung

Die Filmbeschichtung erfolgt analog Beispiel A .

2.3 Präparation der Konjugatschicht 29

<α-Theo>-βGal-Konjugat gelöst in PBS mit 1 % Crotein C wird auf Schablonenpapier (Schöller und Hösch, Gernsheim, BRD) getränkt, so daß 1U Enzymaktivität/cm² zur Verfügung steht.

2.4 Präparation der Substratschicht 34

Wie bei Beispiel A.

2.5 Ergebnisse

30 µl Serum mit verschiedenen Theophyllin-Konzentrationen werden auf Testträger aufgetragen und in dem Gerät "Reflotron" der Anmelderin bei 567 nm vermessen:

| mg/dl Theophyllin | % Remision nach 2 min |
|---|---|
| 0 | 55,10 |
| 1,0 | 38,87 |
| 1,5 | 33,56 |
| 3,1 | 22,84 |

Die erreichbare Abstufung im klinisch relevanten Meßbereich von 1 - 3 mg/dl erlaubt eine sehr gute Meßgenauigkeit.

**Patentansprüche**

1. Testträger zur Analyse einer Probenflüssigkeit mittels einer spezifischen Bindungsreaktion mit einer Mehrzahl von Testschichten, wobei
eine Testschicht (29) ein Konjugat eines Bindungspartners der spezifischen Bindungsreaktion enthält, welches von der Probenflüssigkeit gelöst wird, wobei Komplexe gebildet werden, die ein Maß für den in der Probe enthaltenen Analyt sind, und
eine weitere Testschicht eine Trennschicht (33) ist, die eine poröse Struktur hat und einen Liganden (16) enthält, der eine spezifische Bindungsreaktion mit dem Konjugat macht, um überschüssiges Konjugat aus der in die Trennschicht eindringenden Probenflüssigkeit abzutrennen und die Komplexe, die ein Maß für den in der Probe enthaltenen Analyt sind, passieren zu lassen,
**dadurch gekennzeichnet**, daß
die Trennschicht (33) Trägerpartikel (20) aus anorganischem Material mit Pigmenteigenschaften enthält, an deren Oberfläche reaktive organische Gruppen (12) als Kopplungsgruppen kovalent gebunden sind und der Ligand (16) an die Kopplungsgruppen kovalent gebunden ist, wodurch die Trennschicht (33) zugleich die Funktion einer optischen Sperre erfüllt.

2. Testträger nach Anspruch 1, **dadurch gekennzeichnet**, daß
der Ligand (16) über Brückenmoleküle (13) an die Kopplungsgruppen gebunden ist.

3. Testträger nach Anspruch 1, **dadurch gekennzeichnet**, daß
die Kopplungsgruppen (12) mit Silanisierungsreagenzien eingeführt sind.

4. Testträger nach Anspruch 1, **dadurch gekennzeichnet**, daß
die Trägerpartikel (20) einen mittleren Teilchendurchmesser zwischen $0,2 \mu m$ und $1 \mu m$ haben.

5. Testträger nach Anspruch 1, **dadurch gekennzeichnet**, daß
die Trägerpartikel (20) aus Titandioxid bestehen.

6. Testträger nach einem der Ansprüche 1 - 5, **dadurch gekennzeichnet**, daß
die Trennschicht (33) aus einer Lösung oder Dispersion filmbildender organischer Kunststoffe unter Zusatz eines Öffners in Form von korpuskulären Bestandteilen hergestellt ist.

7. Testträger nach Anspruch 6, **dadurch gekennzeichnet**, daß
der Öffner anorganische Partikel, insbesondere auf Basis von $SiO_2$ mit einem mittleren Teilchendurchmesser von mindestens $5 \mu m$ enthält.

8. Testträger nach Anspruch 7, **dadurch gekennzeichnet**, daß
der Öffner Diatomeenerde enthält.

9. Testträger nach Anspruch 7, **dadurch gekennzeichnet**, daß
die Menge der filmbildenden organischen Kunststoffe maximal 25 Gew% der Gesamtmenge von Trägerpartikeln und Öffner beträgt.

10. Testträger nach Anspruch 7, **dadurch gekennzeichnet**, daß
die Gewichtsrelation zwischen Trägerpartikeln und Öffner zwischen 2:1 und 1:2 liegt.

11. Testträger nach Anspruch 1 oder 7, **dadurch gekennzeichnet**, daß
die Trennschicht auf ein Gewebe (33a) oder Vlies derartig beschichtet ist, daß es teilweise darin eindringt, überwiegend jedoch auf einer Seite verbleibt.

12. Verfahren zur Herstellung einer Trennschicht für einen Testträger nach einem der Ansprüche 1 - 5, **dadurch gekennzeichnet**, daß man
in einem ersten Verfahrensschritt den Liganden mit Hilfe der Kopplungsgruppen an die Trägerpartikel fixiert, in einem zweiten Verfahrensschritt die Partikel insbesondere durch Filtrieren, Zentrifugieren oder Sedimentieren von den zur Fixierung des Liganden notwendigen Substanzen trennt und in einem dritten Verfahrensschritt die Trägerpartikel in einer Trägermatrix, welche die dreidimensionale offene Struktur bildet, anordnet.

13. Verfahren zur Herstellung einer Trennschicht für einen Testträger nach einem der Ansprüche 6 - 11,

**dadurch gekennzeichnet**, daß man in einem ersten Verfahrensschritt den Liganden mit Hilfe der Kopplungsgruppen an die Trägerpartikel fixiert, in einem zweiten Verfahrensschritt die Partikel insbesondere durch Filtrieren, Zentrifugieren oder Sedimentieren von den zur Fixierung des Liganden notwendigen Substanzen trennt und in einem dritten Verfahrensschritt die Trägerpartikel und den Öffner in die Lösung oder Dispersion der filmbildenden organischen Kunststoffe eingemischt und die Mischung zu der Testschicht ausstreicht.

## Claims

1. Test carrier for the analysis of a sample liquid by means of a specific binding reaction with a plurality of test layers, wherein a test layer (29) contains a conjugate of a binding partner of the specific binding reaction which is dissolved by the sample liquid, whereby complexes are formed which are a measure for the analyte contained in the sample and a further test layer is a separating layer (33) which has a porous structure and contains a ligand (16) which makes a specific binding reaction with the conjugate in order to separate excess conjugate from the sample liquid penetrating into the separating layer and allows the complexes, which are measure of the analyte contained in the sample, to pass, characterised in that the separating layer (33) contains carrier particles (20) of inorganic material with pigment properties on the surface of which are covalently bound reactive organic groups (12) as coupling groups and the ligand (16) is covalently bound to the coupling groups, whereby the separating layer (33) simultaneously fulfills the function of an optical barrier.

2. Test carrier according to claim 1, characterised in that the ligand (16) is bound to the coupling groups via bridge molecules (13).

3. Test carrier according to claim 1, characterised in that the coupling groups (12) are introduced with silanisation reagents.

4. Test carrier according to claim 1, characterised in that the carrier particles (20) have an average particle diameter between 0,2 $\mu$m and 1 $\mu$m.

5. Test carrier according to claim 1, characterised in that the carrier particles (20) consist of titanium dioxide.

6. Test carrier according to one of claims 1 - 5, characterised in that the separating layer (33) is produced from a solution or dispersion of film-forming organic synthetic materials with addition of an opener in the form of corpuscular components.

7. Test carrier according to claim 6, characterised in that the opener contains inorganic particles, especially based on $SiO_2$, with an average particle diameter of at least 5 $\mu$m.

8. Test carrier according to claim 7, characterised in that the opener contains diatomaceous earth.

9. Test carrier according to claim 7, characterised in that the amount of the film-forming organic synthetic materials amounts to a maximum of 25 wt.% of the total amount of carrier particles and opener.

10. Test carrier according to claim 7, characterised in that the weight relationship between carrier particles and opener lies between 2:1 and 1:2,

11. Test carrier according to claim 1 or 7, characterised in that the separating layer is coated on to a fabric (33a) or fleece in such a manner that it partly penetrates therein but remains preponderantly on one side.

12. Process for the production of a separating layer for a test carrier according to one of claims 1 - 5, characterised in that, in a first process step, one fixes the ligand on to carrier particles with the help of the coupling groups, in a second process step, separates the particles from the substances necessary for the fixing of the ligand, especially by filtering, centrifuging or sedimentation, and, in a third process step, places the carrier particles in a carrier matrix which forms the three-dimensional, open structure.

13. Process for the production of a separating layer for a test carrier according to one of claims 6 -11, characterised in that, in a first step, one fixes the ligands on to carrier particles with the help of the coupling groups, in a second process step, separates the particles from the substances necessary for the fixing of the ligand, especially by filtering, centrifuging or sedimentation, and, in a third process step, mixes the carrier particles and the opener into the solution or dispersion of the film-forming organic synthetic materials and coats the mixture on to the test layer.

## Revendications

1. Support de test pour analyse d'un échantillon liquide au moyen d'une réaction de liaison spécifique, avec une multiplicité de couches de test, dans lequel

une couche de test (29) contient un conjugué d'un partenaire de liaison de la réaction de liaison spécifique, qui est dissous par l'échantillon liquide, ce qui permet la formation de complexes qui constituent une mesure de l'analyte contenu dans l'échantillon, et

une autre couche de test est une couche de séparation (33) qui présente une structure poreuse et contient un ligand (16) qui entre en réaction de liaison spécifique avec le conjugué, afin de séparer le conjugué en excès de l'échantillon liquide pénétrant dans la couche de séparation et laisser passer les complexes qui constituent une mesure de l'analyte contenu dans l'échantillon,

caractérisé en ce que la couche de séparation (33) contient des particules de support (20) constituées d'une matière inorganique ayant des propriétés de pigment, à la surface desquelles des groupes organiques réactifs (12) sont liés par covalence en tant que groupes de couplage, et le ligand (16) est lié par covalence aux groupes de couplage, ce qui permet à la couche de séparation (33) de remplir également la fonction d'une barrière optique.

2. Support de test selon la revendication 1, caractérisé en ce que le ligand (16) est lié aux groupes de couplage par l'intermédiaire de molécules de pontage (13).

3. Support de test selon la revendication 1, caractérisé en ce que les groupes de couplage (12) sont introduits avec des réactifs de silanisation.

4. Support de test selon la revendication 1, caractérisé en ce que les particules de support (20) présentent un diamètre moyen de particules compris entre 0,2 $\mu$m et 1 $\mu$m.

5. Support de test selon la revendication 1, caractérisé en ce que les particules de support (20) sont constituées de dioxyde de titane.

6. Support de test selon l'une quelconque des revendications 1-5, caractérisé en ce que la couche de séparation (33) est préparée à partir d'une solution ou dispersion de matière synthétique organique filmogène, additionnée d'un élément ouvreur sous la forme de constituants corpusculaires.

7. Support de test selon la revendication 6, caractérisé en ce que l'élément ouvreur contient des particules inorganiques, en particulier à base de $SiO_2$, ayant un diamètre moyen de particules d'au moins 5 $\mu$m.

8. Support de test selon la revendication 7, caractérisé en ce que l'élément ouvreur contient de la terre d'infusoire.

9. Support de test selon la revendication 7, caractérisé en ce que la quantité de matière synthétique organique filmogène constitue au maximum 25 % en poids de la quantité totale des particules de support et de l'élément ouvreur.

10. Support de test selon la revendication 7, caractérisé en ce que la relation pondérale entre les particules de support et l'élément ouvreur est comprise entre 2 : 1 et 1 : 2.

11. Support de test selon la revendication 1 ou 7, caractérisé en ce que la couche de séparation est appliquée sur un tissu (33a) ou un non-tissé de façon à y pénétrer partiellement, en restant toutefois principalement sur une face.

12. Procédé de préparation d'une couche de séparation pour un support de test selon l'une quelconque des revendications 1-5, caractérisé en ce que dans une première étape du procédé, on fixe le ligand sur les particules de support à l'aide des groupes de couplage, dans une deuxième étape du procédé, on sépare les particules, en particulier par filtration, centrifugation ou sédimentation d'avec les substances nécessaires à la fixation du ligand, et dans une troisième étape du procédé, on dispose les particules de support dans une matrice de support qui forme une structure ouverte tridimensionnelle.

13. Procédé de préparation d'une couche de séparation pour un support de test selon l'une quelconque des revendications 6-11, caractérisé en ce que dans une première étape du procédé, on fixe le ligand sur les particules de support à l'aide des groupes de couplage, dans une deuxième étape du procédé, on sépare les particules, en particulier par filtration, centrifugation ou sédimentation, d'avec les substances nécessaires à la fixation du ligand, et dans une troisième étape du procédé, on incorpore les particules de support et l'élément ouvreur dans la solution ou dispersion de la matière organique filmogène et on étale le mélange pour produire la couche de test.

# FIG. 1

# FIG. 2

# FIG.3